# EUROPÄISCHE PATENTSCHRIFT

(11) **EP 3 469 051 B1**
(45) Veröffentlichungstag und Bekanntmachung des Hinweises auf die Patenterteilung: **27.10.2021**
(21) Anmeldenummer: 17733987.6
(22) Anmeldetag: 09.06.2017
(51) Int. Cl.: C11D 3/395, C11D 3/04, A61L 2/00, C11D 17/04, C11D 3/48, D21H 21/36

(54) **CHLORDIOXID DESINFEKTIONSTUCH**
CHLORINE DIOXIDE DISINFECTING CLOTH
LINGETTE DÉSINFECTANTE À BASE DE DIOXYDE DE CHLORE

(30) Priorität: 10.06.2016 DE 102016007081
(43) Veröffentlichungstag der Anmeldung: 17.04.2019
(73) Patentinhaber: Alethia Life Sciences AG, 9443 Widnau (CH)
(72) Erfinder: OBERWALDER, Sebastian, 81371 München (DE)
(74) Vertreter: Kiwit, Benedikt
(86) Internationale Anmeldenummer: PCT/EP2017/064101
(87) Internationale Veröffentlichungsnummer: WO 2017/212024

(56) Entgegenhaltungen:
- EP-A2- 0 423 817
- WO-A1-2008/065551
- WO-A1-2015/191811
- WO-A1-2016/102606
- US-A1- 2006 051 266

## Beschreibung

Die vorliegende Erfindung betrifft hochwirksame Desinfektions- bzw. Sterilisationstücher.

Es besteht ein großer Bedarf nach hochwirksamen Desinfektionstüchern und Sterilisationstüchern, insbesondere im Labor- und Medizinbereich. Musterbeispiele dafür sind die Sterilisation von medizinischen Geräten, die nicht autoklaviert werden können, wie z.B. Endoskope sowie die Reinigung von Medizintechnik- und Laborgeräten. Bei der Reinigung von solchen Geräten sind herkömmliche Desinfektionstücher aufgrund der eingeschränkten bzw. langsamen Wirksamkeit der verwendeten Desinfektionsmittel oft ungeeignet. Dies gilt insbesondere für das wohl am weitesten verwendete Desinfektionsmittel Alkohol, welches eine relativ lange Einwirkzeit benötigt, die aber aufgrund von Verdunstung in der Regel nicht erreicht wird.

Soweit bekannt erreicht bisher nur ein einziges System eine zufriedenstellende Wirksamkeit, indem es als Wirkstoff Chlordioxid einsetzt, siehe z.B. WO2005107823 sowie US2014142489 und WO2015040366. Aufgrund der geringen Stabilität des aktiven Wirkstoffs Chlordioxid arbeitet das dort beschriebene System damit, den Wirkstoff unmittelbar bei Anwendung dadurch herzustellen, dass die vorher imprägnierten Wischtücher mit einem Aktivator-Spray eingesprüht und somit aktiviert werden. Dabei wird das benötigte Chlordioxid unmittelbar vor Anwendung hergestellt, weshalb die Problematik der Lagerstabilität weitgehend gelöst wird. Die Verwendung eines manuell zu bedienenden Aktivator-Sprays führt allerdings wiederum zu einem anderen Problem, nämlich der Schwierigkeit der korrekten Dosierung des Aktivators. Dieses Problem kann Aufgrund der Variabilität bei der manuellen Bedienung nur dadurch einigermaßen gelöst werden, dass der Aktivator grundsätzlich überdosiert wird. Das führt jedoch wiederum dazu, dass signifikante Reste des Aktivators auf der desinfizierten Fläche verbleiben, weshalb ein Nachreinigen dieser Flächen erforderlich wird, um den überschüssigen Aktivator zu entfernen.

Das stellt jedoch aus mehreren Gründen ein signifikantes Problem dar: Insbesondere kann es beim Nachreinigungs-Vorgang mit einem nunmehr nicht mehr sterilisierenden Tuch zu erneuten Verunreinigungen kommen, wodurch der Zweck der vorangehenden Sterilisation vereitelt wird und eine sichere Anwendung des Systems eigentlich nicht mehr gewährleistet ist. Ebenso ist ein weiterer Arbeitsschritt notwendig, was neben neuen Fehlerquellen vor allem einen nicht unerheblichen zusätzlichen Zeitaufwand sowie Zusatzkosten sowohl hinsichtlich Material und Arbeitszeit nach sich zieht.

EP 0 423 817 A2 offenbart einen Chlordioxidgenerator, der Chlordioxid-Gas erzeugt und in die Atmosphäre abgibt. WO 2008/065551 A1 offenbart ein Wischtuch, das aus mehreren Tuchlagen besteht, zwischen denen mehrere, jeweils mit unterschiedlichen Flüssigkeiten gefüllte Blasen ("blisters") angeordnet sind, so dass nach Zerdrücken der Blasen beispielsweise Chlordioxid durch die Reaktion der jeweiligen Flüssigkeiten erzeugt werden kann.

Die vorliegende Erfindung löst das Problem dadurch, dass das Desinfektionstuch, welches mit einer für die Herstellung von Chlordioxid notwendigen Komponente imprägniert ist, durch in Kontakt bringen des Desinfektionstuchs mit einer als Einzeldosis vorabgepackten klar definierten Menge eines flüssig vorliegenden Aktivierungsmittels aktiviert wird. Dadurch lassen sich die Mengen der Imprägnierung und des Aktivators jeweils genau im erforderlichen stöchiometrischen Verhältnis abstimmen, bzw. kann der Überschuss einer Komponente derart gering ausfallen, dass eine Nachreinigung zur Entfernung des Überschusses einer Komponente nicht mehr erforderlich ist.

Insbesondere stellt die vorliegende Erfindung ein Desinfektionstuch bereit, das vor Verwendung durch in Kontakt bringen eines
A.) mit einem Chlorit- oder Chlorat-Salz, bevorzugt Natriumchlorit, Natriumchlorat, Kaliumchlorit, Kaliumchlorat, Ammoniumchlorit oder Ammoniumchlorat imprägnierten Tuchs mit
B.) einer als Einzeldosis vorabgepackten klar definierten Menge eines geeigneten flüssigen bzw. gelösten bzw. in einer Flüssigkeit beigemischten Aktivierungsmittels zur Erzeugung von Chlordioxid, bevorzugt Salzsäure, Peroxodischwefelsäure, Phosphorsäure oder einem Oxidationsmittel aktiviert wird, wobei ein oder mehrere imprägnierte Tücher und die Aktivierungsflüssigkeit in einer gemeinsamen Verpackungseinheit verpackt sind und die Aktivierung durch Öffnen und/oder Durchbrechen und/oder Auflösen einer Barriere, welche das Tuch bzw. die Tücher und die Aktivierungsflüssigkeit trennt, erfolgt. Dabei ist das erfindungsgemäße Desinfektionstuch dadurch gekennzeichnet, dass die Verpackung ein Formteil mit zwei oder mehr voneinander durch aufhebbare Barrieren getrennten Verpackungsräumen ist, welcher sich durch einfachen Druck oder Zug in eine Richtung in ein Formteil mit nur einer Kammer umwandeln lässt.

Als Imprägnierung für das Tuch kommen grundsätzlich alle Chlorit- oder Chlorat-Salze in Betracht, bevorzugt Natriumchlorit, Natriumchlorat, Kaliumchlorit, Kaliumchlorat, Ammoniumchlorit oder Ammoniumchlorat. Als Aktivierungsmittel zur Erzeugung von Chlordioxid sind grundsätzlich alle bekannten Reaktionspartner zur Erzeugung von Chlordioxid geeignet, bevorzugt ein Mittel der

### Gruppe 1:

Säuren, weiter bevorzugt: Salzsäure, Schwefelsäure, Phosphorsäure, Peroxyessigsäure, Carbonsäuren mit einem organischen Rest von C1-C40;
Persulfate;
Peroxodisulfate;
Perborate;
Percarbonate;
Peroxodicarbonate;
Permanganate, weiter bevorzugt Kaliumpermanganat;
Hypochlorite, weiter bevorzugt Natriumhypochlorit, Kaliumhypochlorit;
Iod
und/oder Wasserstoffperoxid
besonders bevorzugt Salzsäure, Peroxodischwefelsäure, Phosphorsäure oder Wasserstoffperoxid.

Statt einem können auch mehrere Tücher in derselben Packung verpackt sein, z.B. 50 - 500 Tücher in einer Hartkunststoffverpackung mit Entnahmeöffnung (Trommel/Eimer/Spenderdose).

In einer anderen bevorzugten Ausführungsform sind die Aktivierungsflüssigkeit und das imprägnierte Tuch gemeinsam in einer Kunststoffwanne verpackt, aber durch eine entfernbare bzw. durchstoßbare Barriere getrennt. Diese Wanne ist am oberen Ende mit einer flüssigkeitsdichten Folie ver schlossen. Die Trennbarriere kann nun beim Abziehen der flüssigkeitsdichten Verschlussfolie mit abgezogen werden, wodurch dann die Aktivierungsflüssigkeit zum imprägnierten Tuch fließt, von diesem Aufgesogen wird und somit die Reaktion der Komponenten zu Chlordioxid stattfindet. Alternativ kann die Barriere im geschlossenen Zustand durchstoßen werden, beispielsweise durch Druck auf die Verschlussfolie, wodurch das Zusammenfließen und Aufsaugen bzw. die Reaktion der Komponenten zu Chlordioxid noch in der geschlossenen Verpackung stattfindet.

In der vorliegenden Erfindung ist die Verpackung ein Formteil mit zwei voneinander getrennten Verpackungsräumen, welche sich durch einfachen Druck oder Zug in eine Richtung in ein Formteil mit nur einer Kammer umwandeln. Hierbei befindet sich das imprägnierte Tuch in der einen Kammer und die Aktivierungsflüssigkeit in der anderen Kammer. Wird das Formteil durch Druck oder Zug dann zu einer einheitlichen Kammer geformt, kommen Tuch und Aktivator in Kontakt, und die Reaktion zu Chlordioxid findet statt. Der Formteil kann vor Verformung durch Druck oder Zug auch mehr als zwei Kammern enthalten, was vorteilhaft sein kann, wenn neben der Chemikalie zur Erzeugung von Chlordioxid noch weitere Komponenten auf das Tuch in situ kurz vor Anwendung aufgebracht werden sollen.

In einer besonders bevorzugten Ausführungsform ist der soeben beschriebene Formteil in Kunststoff ausgeführt, bevorzugt PE (Polyethylen), PP (Polypropylen), PA (Polyamid), PVC (Polyvinylchlorid), PTFE (Polytetrafluorethylen) oder PU (Polyurethan), und an einer Seite nur mit einer abziehbaren oder durchstoßbaren Membrane, bevorzugt ebenfalls aus einem der genannten Kunststoffe oder aus Papier ausgeführt, verschlossen.

In einer weiter bevorzugten Ausführung ist im soeben beschriebenen mit einer Membran verschlossenen Kunststoff-Formteil ein zusammengepresstes trockenes Desinfektionstuch aus einem saugfähigen Material, bevorzugt Zellulose, verpackt, welches durch Kontakt mit der Aktivatorflüssigkeit sein Volumen im Vergleich zur Trockenlagerung um mindestens 50% erhöht. Weiter bevorzugt expandiert das derart vergrößerte Desinfektionstuch damit über die Verpackung hinaus und kann somit leicht entnommen werden.

Zur besseren Steuerung der Reaktion enthalten das Desinfektionstuch und/oder die Aktivierungsflüssigkeit in einer bevorzugten Ausführungsform zusätzlich eine oder mehrere den pH-Wert regelnden Substanzen, bevorzugt ein Carbonat, Hydrogencarbonat, Sulfat, Hydrogensulfat, Phosphat und/oder Hydrogenphosphat, entweder als Beimischung zur Flüssigkeit und/oder als zusätzliche Imprägnierung. Aus Handhabungsgründen wird eine Beimischung zur Flüssigkeit bevorzugt, wobei zur Verbesserung der Mischung bzw. Verteilung auch eine oder mehrere Substanzen in der Flüssigkeit vorliegen können, während eine oder mehrere Substanzen alternativ oder zusätzlich als Imprägnierung verwendet werden können.

In einer weiteren bevorzugten Ausführungsform enthalten das Desinfektionstuch und/oder die Aktivierungsflüssigkeit zusätzlich ein oder mehrere Tenside, bevorzugt eine quaternäre Ammoniumverbindung oder ein nichtorganisches Tensid, entweder als Beimischung zur Flüssigkeit und/oder als zusätzliche Imprägnierung. Aus Handhabungsgründen wird hier ebenfalls eine Beimischung zur Flüssigkeit bevorzugt, wobei zur Verbesserung der Mischung bzw. Verteilung auch eine oder mehrere Substanzen in der Flüssigkeit vorliegen können, während eine oder mehrere Substanzen alternativ oder zusätzlich als Imprägnierung verwendet werden können. Die genannten Substanzen können die Desinfektionswirkung weiter erhöhen und/oder die Verteilung des Chlordioxids auf dem zu desinfizierenden Gegenstand bzw. der zu desinfizierenden Oberfläche verbessern. Ebenso können die Tenside das Entweichen des Chlordioxids aus der Flüssigkeit verlangsamen, und somit dazu beitragen eine etwaige Umweltbelastung bzw. Gefährdung des Anwenders zu vermeiden bzw. zu reduzieren. Dadurch dass die Substanzen nur kurzzeitig mit Chlordioxid in Kontakt kommen, weil dieses erst kurz vor Anwendung durch Kontakt der Aktivierungsflüssigkeit mit der Imprägnierung erzeugt wird, ist die Wechselwirkung dieser Substanzen mit dem Chlordioxid nur gering. Insbesondere kommt es nur zu einem geringfügigen gegenseitigen Abbau, weshalb die gemeinsame Anwendung dieser Substanzen mit Chlordioxid vorliegend unproblematisch möglich ist.

In einer weiteren bevorzugten Ausführungsform enthalten das Desinfektionstuch und/oder die Aktivierungsflüssigkeit zusätzlich eine oder mehrere Substanzen der folgenden

### Gruppe 2:

Guanidiniumderivate, insbesondere PHMB;
Aldehyde, insbesondere Glutaraldehyd;
Phenole, insbesondere Chlorxylenol, Triclosan;
Chlorhexidin;
Octenidin;
Phenoxyethanole;
und/oder Phosphatester, bevorzugt ethoxylierte aliphatische Phosphatester bzw. Phosphatdiester und/oder alkylether Phosphate
entweder als Beimischung zur Flüssigkeit und/oder als zusätzliche Imprägnierung. Aus Handhabungsgründen wird hier ebenfalls eine Beimischung zur Flüssigkeit bevorzugt, wobei zur Verbesserung der Mischung bzw. Verteilung auch eine oder mehrere Substanzen in der Flüssigkeit vorliegen können, während eine oder mehrere Substanzen alternativ oder zusätzlich als Imprägnierung verwendet werden können. Die genannten Substanzen können die Desinfektionswirkung weiter erhöhen und/oder die Verteilung des Chlordioxids auf dem zu desinfizierenden Gegenstand bzw. der zu desinfizierenden Oberfläche verbessern und/oder neben der kurzfristigen Desinfektionswirkung für eine zusätzliche mittel- bzw. langfristige antimikrobielle Wirksamkeit sorgen. Dadurch dass die Substanzen nur kurzzeitig mit Chlordioxid in Kontakt kommen, weil dieses erst kurz vor Anwendung durch Kontakt der Aktivierungsflüssigkeit mit der Imprägnierung erzeugt wird, und danach relativ schnell von der Oberfläche verdampft bzw. ausgast, ist die Wechselwirkung dieser Substanzen mit dem Chlordioxid nur gering. Insbesondere kommt es nur zu einem geringfügigen gegenseitigen Abbau, weshalb die gemeinsame Anwendung dieser Substanzen mit Chlordioxid vorliegend unproblematisch möglich ist.

In einer weiteren bevorzugten Ausführungsform enthält die Aktivierungsflüssigkeit als Lösemittel und/oder als zusätzlichen Inhaltsstoff eine oder mehrere Substanzen der nachfolgenden

### Gruppe 3:

Wasser;
Alkohole, insbesondere Ethanol und Isopropanol;
Ketone, insbesondere Aceton;
Sulfoxide, insbesondere DMSO;
und/oder Carbonsäureester, insbesondere Essigsäureethylester

Diese Substanzen führen vor allem zu einer besseren Lösung und Verteilung der Inhaltsstoffe. Daneben könne sie auch die Verteilung des Chlordioxids auf dem zu desinfizierenden Gegenstand bzw. der zu desinfizierenden Oberfläche verbessern und/oder die Desinfektionswirkung weiter erhöhen. Weiters können sie die Eindringtiefe des Desinfektionsmittels in die behandelte Oberfläche erhöhen. Dadurch dass die Substanzen nur kurzzeitig mit Chlordioxid in Kontakt kommen, weil dieses erst kurz vor Anwendung durch Kontakt der Aktivierungsflüssigkeit mit der Imprägnierung erzeugt wird, ist die Wechselwirkung dieser Substanzen mit dem Chlordioxid nur gering. Insbesondere kommt es nur zu einem geringfügigen gegenseitigen Abbau, weshalb die gemeinsame Anwendung dieser Substanzen mit Chlordioxid vorliegend unproblematisch möglich ist.

Zusätzlich kann die Aktivierungsflüssigkeit einen Farbindikator enthalten, durch welchen das Desinfektionstuch bei Kontakt seine Farbe verändert. Hierfür kann das Desinfektionstuch ganz oder teilweise unter einer transparenten Verpackungsoberfläche verpackt werden. Die Indikatorsubstanz kann auch als Imprägnierung im Desinfektionstuch enthalten sein, und bei Kontakt mit Flüssigkeit aktiviert werden. Besonders bevorzugt ist die Indikatorflüssigkeit gleichzeitig ein chemischer Indikator für das Vorliegen von Chlordioxid, z.B. in Schwefelsäure gelöstes Kaliumjodid, bei dem es bei Kontakt mit Chlordioxid zu einem Farbumschlag kommt (Iodid-Iod Reaktion).

Bevorzugt wird das oben beschriebene Desinfektionstuch, gleich in welcher Ausführung zur Desinfektion und/oder Sterilisation von medizinischen Instrumenten und/oder Medizinprodukten und/oder Laborgeräten und/oder Oberflächen, bevorzugt in: Krankenhäusern und/oder Arztpraxen und/oder Labors, insbesondere Labors mit Sicherheitsstufe 2 oder höher verwendet.

## Patentansprüche

1. Desinfektionstuch, das vor Verwendung durch in Kontakt bringen eines
A.) mit einem Chlorit- oder Chlorat-Salz, bevorzugt Natriumchlorit, Natriumchlorat, Kaliumchlorit, Kaliumchlorat, Ammoniumchlorit oder Ammoniumchlorat imprägnierten Tuchs
mit
B.) einer als Einzeldosis vorabgepackten klar definierten Menge eines geeigneten flüssigen bzw. gelösten bzw. in einer Flüssigkeit beigemischten Aktivierungsmittels zur Erzeugung von Chlordioxid, bevorzugt Salzsäure, Peroxodischwefelsäure, Phosphorsäure oder einem Oxidationsmittel
aktiviert wird;
wobei ein oder mehrere imprägnierte Tücher und die Aktivierungsflüssigkeit in einer gemeinsamen Verpackungseinheit verpackt sind und die Aktivierung durch Öffnen und/oder Durchbrechen und/oder Auflösen einer Barriere, welche das Tuch bzw. die Tücher und die Aktivierungsflüssigkeit trennt, erfolgt;
**dadurch gekennzeichnet,**
**dass** die Verpackung ein Formteil mit zwei oder mehr voneinander durch aufhebbare Barrieren getrennten Verpackungsräumen ist, welcher sich durch einfachen Druck oder Zug in eine Richtung in ein Formteil mit nur einer Kammer umwandeln lässt.

2. Desinfektionstuch nach Anspruch 1, **dadurch gekennzeichnet, dass** der aus Druck oder Zug resultierende Ein-Kammer-Formteil an einer Seite eine leicht zu öffnende oder zu durchstoßende Verpackungs-Membran enthält.

3. Desinfektionstuch nach Anspruch 1 oder 2, **dadurch gekennzeichnet, dass** das Desinfektionstuch aus einem saugfähigen Material besteht welches trocken in gefalteter und/oder gepresster Form vorliegt und durch Kontakt mit einer Flüssigkeit, bevorzugt der Aktivierungsflüssigkeit B.), sein Volumen um mindestens 50% im Vergleich zur Trockenlagerung erhöht.

4. Desinfektionstuch nach einem der vorangehenden Ansprüche, **dadurch gekennzeichnet, dass** die Aktivierungsflüssigkeit zusätzlich eine oder mehrere den pH-Wert regelnden Substanzen enthält, bevorzugt ein Carbonat, Hydrogencarbonat, Sulfat, Hydrogensulfat, Phosphat und/oder Hydrogenphosphat, und/oder das Desinfektionstuch zusätzlich mit einer oder mehreren den pH-Wert regelnden Substanzen, bevorzugt den zuvor genannten, imprägniert ist.

5. Desinfektionstuch nach einem der vorangehenden Ansprüche, **dadurch gekennzeichnet, dass** entweder die Aktivierungsflüssigkeit zusätzlich ein oder mehrere Tenside enthält, bevorzugt eine quaternäre Ammoniumverbindung und/oder ein nichtorganisches Tensid, und/oder das Desinfektionstuch zusätzlich mit einem oder mehreren Tensiden, bevorzugt den zuvor genannten, imprägniert ist.

6. Desinfektionstuch nach einem der vorangehenden Ansprüche, **dadurch gekennzeichnet, dass** entweder die Aktivierungsflüssigkeit zusätzlich eine oder mehrere Substanzen der folgenden
Gruppe 2:
Guanidiniumderivate, insbesondere PHMB;
Aldehyde, insbesondere Glutaraldehyd;
Phenole, insbesondere Chlorxylenol, Triclosan;
Chlorhexidin;
Octenidin;
Phenoxyethanole;
und/oder Phosphatester, bevorzugt ethoxylierte aliphatische Phosphatester bzw.
Phosphatdiester und/oder alkylether Phosphate
enthält, und/oder das Desinfektionstuch stattdessen bzw. zusätzlich mit einer oder mehreren Substanzen dieser Gruppe 2 imprägniert ist.

7. Desinfektionstuch nach einem der vorangehenden Ansprüche, **dadurch gekennzeichnet, dass** das Aktivierungsmittel in einer Substanz aus der folgenden
Gruppe 3:
Wasser;
Alkohole, insbesondere Ethanol und Isopropanol;
Ketone, insbesondere Aceton;
Sulfoxide, insbesondere DMSO;
und/oder Carbonsäureester, insbesondere Essigsäureethylester
gelöst und/oder mit dieser gemischt ist.

8. Verwendung eines Desinfektionstuchs nach einem der vorangehenden Ansprüche zur Sterilisation von medizinischen Instrumenten und/oder Medizinprodukten und/oder Oberflächen.

## Claims

1. Disinfectant cloth, which is activated prior to use by contacting
A.) a cloth, impregnated with a chlorite salt or a chlorate salt, preferably sodium chlorite, sodium chlorate, potassium chlorite, potassium chlorate, ammonium chlorite or ammonium chlorate,
with
B.) a clearly defined amount of an appropriate activating agent, liquid and / or dissolved and / or mixed in a liquid, prepackaged as a single dose, for the production of chlorine dioxide, preferably hydrochloric acid, peroxodisulphuric acid, phosphoric acid or an oxidizing agent;
wherein one or more impregnated cloths and the activating liquid are packaged in a common packaging unit, and the activation occurs by opening and / or breaking and / or dissolving a barrier which separates the cloth and / or the cloths and the activating liquid ;
**characterised in**
**that** the packaging is a moulded part with two or more packaging compartments separated from each other by removable barriers which can be converted into a moulded part with only one chamber by simple pressure or pull in one direction.

2. Disinfectant cloth according to claim 1, **characterised in that** the one chamber moulded part resulting from pressure or pull on one side contains a packaging membrane that is easy to open or puncture.

3. Disinfectant cloth according to claim 1 or 2, **characterised in that** the disinfectant cloth is made of an absorbent material, which is available dry in folded and / or pressed form, and increases its volume by at least 50 % compared with dry storage through contact with a liquid, preferably the activating liquid B.).

4. Disinfectant cloth according to any one of the preceding claims, **characterised in that** the activating liquid in addition contains one or more substances regulating the pH value, preferably a carbonate, hydrogen carbonate, sulphate, hydrogen sulphate, phosphate and / or hydrogen phosphate, and / or the disinfectant cloth in addition is impregnated with one or more substances regulating the pH value, preferably the substances mentioned above.

5. Disinfectant cloth according to any one of the preceding claims, **characterised in that** either the activating liquid contains one or more surfactants in addition, preferably quaternary ammonium compounds and / or a non organic surfactant, and / or the disinfectant cloth is impregnated in addition with one or more surfactants, preferably the substances mentioned above.

6. Disinfectant cloth according to any one of the preceding claims, **characterised in that** either the activating liquid in addition contains one or more substances of the following
group 2 :
guanidinium derivatives, in particular PHMB ;
aldehydes, in particular glutaraldehyde ;
phenols, in particular chloroxylenol, triclosan ;
chlorhexidine ;
octenidine ;
phenoxyethanols ;
and / or phosphate esters, preferably ethoxylated aliphatic phosphate esters and / or phosphate diesters and / or alkyl ether phosphates, and / or the disinfectant cloth is impregnated instead and / or in addition with one or more substances of this group 2.

7. Disinfectant cloth according to any one of the preceding claims, **characterised in that** the activating agent is dissolved in and / or mixed with a substance from the following
group 3 :
water;
alcohols, in particular ethanol and isopropanol;
ketones, in particular acetone ;
sulfoxides, in particular DMSO ;
and / or carboxylic acid ester, in particular ethyl acetate.

8. Use of a disinfectant cloth according to any one of the preceding claims for sterilisation of medical instruments and / or medical devices and / or surfaces.

## Revendications

1. Lingette désinfectante, qui est activée avant l'utilisation par mise en contact d'une
A.) lingette imprégnée avec un sel de chlorite ou chlorate, de préférence chlorite de sodium, chlorate de sodium, chlorite de potassium, chlorate de potassium, chlorite d'ammonium ou chlorate d'ammonium
avec
B.) une quantité définie clairement emballée au préalable comme dose individuelle d'un moyen d'activation approprié liquide ou dissous ou mélangé dans un liquide pour la production de dioxyde de chlore, de préférence acide chlorhydrique, acide peroxydisulfurique, acide phosphorique ou un moyen d'oxydation ;
dans laquelle une ou plusieurs lingettes imprégnées et le liquide d'activation sont emballés dans une unité d'emballage commune et l'activation est effectuée par ouverture et/ou rupture et/ou dissolution d'une barrière qui sépare la lingette ou les lingettes et le liquide d'activation ;
**caractérisée en ce**
**que** l'emballage est une pièce moulée avec deux espaces d'emballage ou plus séparés les uns des autres par des barrières pouvant être levées, laquelle peut être transformée par simple pression ou traction dans une direction en une pièce moulée avec une seule chambre.

2. Lingette désinfectante selon la revendication 1, **caractérisée en ce que** la pièce moulée à une chambre résultant de la pression ou traction contient au niveau d'un côté une membrane d'emballage à ouvrir légèrement ou à transpercer.

3. Lingette désinfectante selon la revendication 1 ou 2, **caractérisée en ce que** la lingette désinfectante se compose d'un matériau absorbant qui se présente au sec sous la forme pliée et/ou pressée et augmente par contact avec un liquide, de préférence du liquide d'activation B.), son volume d'au moins 50 % par rapport au stockage au sec.

4. Lingette désinfectante selon l'une des revendications précédentes, **caractérisée en ce que** le liquide d'activation contient en outre une ou plusieurs substances régulant la valeur pH, de préférence un carbonate, carbonate d'hydrogène, sulfate, sulfate d'hydrogène, phosphate et/ou phosphate d'hydrogène, et/ou la lingette désinfectante est imprégnée en outre avec une ou plusieurs substances régulant la valeur pH, de préférence celles citées précédemment.

5. Lingette désinfectante selon l'une des revendications précédentes, **caractérisée en ce que** le liquide d'activation contient en outre un ou plusieurs tensio-actifs, de préférence un composé d'ammonium quaternaire et/ou un tensio-actif non-organique, et/ou la lingette désinfectante est en outre imprégnée avec un ou plusieurs tensio-actifs, de préférence ceux cités précédemment.

6. Lingette désinfectante selon l'une des revendications précédentes, **caractérisée en ce que** le liquide d'activation contient en outre une ou plusieurs substances du groupe 2 suivant :
dérivés de guanidinium, en particulier PHMB ;
aldéhydes, en particulier glutaraldéhyde ;
phénols, en particulier chloroxylénol, triclosan ;
chlorhéxidine ;
octénidine ;
phénoxyéthanoles ;
et/ou ester de phosphate, de préférence ester de phosphate aliphatique éthoxylé ou diester de phosphate et/ou phosphates d'alkyléther, et/ou la lingette désinfectante est imprégnée à la place ou en outre avec une ou plusieurs substances de ce groupe 2.

7. Lingette désinfectante selon l'une des revendications précédentes, **caractérisée en ce que** le moyen d'activation est dissous dans une substance du groupe 3 suivant :
eau ;
alcools, en particulier éthanol et isopropanol ;
cétones, en particulier acétone ;
sulfoxydes, en particulier DMSO ;
et/ou ester d'acide carboxylique, en particulier acétate d'éthyle
et/ou mélangé avec celle-ci.

8. Utilisation d'une lingette désinfectante selon l'une des revendications précédentes pour la stérilisation d'instruments médicaux et/ou produits médicaux et/ou surfaces.
